# EUROPEAN PATENT APPLICATION

(11) **EP 4 086 634 A1**
(43) Date of publication of application: **09.11.2022**
(21) Application number: 21172527.0
(22) Date of filing: 06.05.2021
(51) Int. Cl.: G01N 33/86

(54) **METHOD FOR DIAGNOSING A PREDISPOSITION OF A LIVING BEING TO DEVELOP THROMBOCYTOPENIA**

(71) Applicant: Eberhard Karls Universität Tübingen Medizinische Fakultät, 72074 Tübingen (DE)
(72) Inventor: Bakchoul, Tamam, 72072 Tübingen (DE); Althaus, Karina, 72127 Kusterdingen-Immenhausen (DE); Zlamal, Jan, 72119 Ammerbuch (DE)
(74) Representative: Witte, Weller & Partner Patentanwälte mbB

(57) **Abstract**

The present invention relates to a method for diagnosing a predisposition of a living being to develop thrombocytopenia, and uses associated therewith.

## Description

The present invention relates to a method for diagnosing a predisposition of a living being to develop thrombocytopenia or a suffering therefrom, and uses associated therewith.

### FIELD OF THE INVENTION

The present invention relates to the field of molecular medicine, more particular to the field of molecular diagnostics, preferably to the diagnostics of molecular blood markers associated with thrombocytopenia.

### BACKGROUND OF THE INVENTION

Thrombocytopenia is a condition characterized by abnormally low levels of platelets, also known as thrombocytes, in the blood. It is the most common coagulation disorder among intensive care patients and is seen in 20% of medical patients and a third of surgical patients.

A normal human platelet count ranges from 150,000 to 450,000 platelets per microliter of blood. Values outside this range do not necessarily indicate disease. One common definition of thrombocytopenia requiring emergency treatment is a platelet count below 50,000 per microliter. Thrombocytopenia can be contrasted with the conditions associated with an abnormally high level of platelets in the blood: thrombocythemia (when the cause is unknown), and thrombocytosis (when the cause is known).

Heparin-induced thrombocytopenia (HIT) is the development of thrombocytopenia due to the administration of various forms of heparin, an anticoagulant. HIT predisposes to thrombosis, i.e. the abnormal formation of blood clots inside a blood vessel, because platelets release microparticles that activate thrombin, thereby leading to thrombosis. When thrombosis is identified the condition is called heparin-induced thrombocytopenia and thrombosis (HITT). HIT is caused by the formation of abnormal antibodies that activate platelets. If someone receiving heparin develops new or worsening thrombosis, or if the platelet count falls, HIT can be confirmed with specific blood tests.

HIT may be suspected if blood tests show a falling platelet count in someone receiving heparin, even if the heparin has already been discontinued. Professional guidelines recommend that people receiving heparin have a complete blood count which includes a platelet count, on a regular basis while receiving heparin. However, not all people with a falling platelet count while receiving heparin turn out to have HIT. The timing, severity of the thrombocytopenia, the occurrence of new thrombosis, and the presence of alternative explanations, all determine the likelihood that HIT is present.

A commonly used score to predict the likelihood of HIT is the "4 Ts" score introduced in 2003. A score of 0-8 points is generated; if the score is 0-3, HIT is unlikely. A score of 4-5 indicates intermediate probability, while a score of 6-8 makes it highly likely. Those with a high score may need to be treated with an alternative drug, while more sensitive and specific tests for HIT are performed, while those with a low score can safely continue receiving heparin, as the likelihood that they have HIT is extremely low. While the 4T score is a laborious procedure that is difficult to implement into daily diagnostic routines, is also is quite unreliable in its prognostic value.

Vaccine-induced immune thrombotic thrombocytopenia (VITT), also termed vaccine-induced prothrombotic immune thrombocytopenia (VIPIT) are rare types of blood clotting events that were initially observed in a very small number of people who had previously received the Oxford-AstraZeneca COVID-19 vaccine (AZD1222 or ChAdOx1, respectively) during the COVID-19 pandemic. It was subsequently also described in the Johnson & Johnson COVID-19 vaccine leading to suspension of its use until its safety had been reassessed. In April 2021, AstraZeneca and the EMA updated their information for healthcare professionals about ChAdOx1, saying it was "considered plausible" that there was a causal relationship between the vaccination and the occurrence of thrombosis in combination with thrombocytopenia and that, "although such adverse reactions are very rare, they exceeded what would be expected in the general population. On 7 April 2021 the EMA noted one "plausible explanation" for the combination of blood clots and low blood platelets is "an immune response, leading to a condition similar to one seen sometimes in patients treated with heparin", that is heparin induced thrombocytopenia (HIT).

Currently, no method is available that allows a treating physician to identify in advance whether a patient who should urgently receive a vaccination, for example by using a vector vaccine, has a tendency to develop thrombosis or thrombocytopenia and should therefore be switched to a different vaccine if necessary.

Against this background it is an object underlying the invention to provide a new method for diagnosing a predisposition of a living being to develop thrombocytopenia and/or for diagnosing a living of suffering from thrombocytopenia, by means of which the disadvantages of the methods of the art are avoided or at least reduced. In particular, a method should be provided which is of low complexity and provides results in a reliable and reproducible manner.

The present invention satisfies these and other needs.

### SUMMARY OF THE INVENTION

The present invention provides for a method for diagnosing a predisposition of a living being to develop thrombocytopenia, comprising the following steps:
1. Providing blood serum of a living being to be diagnosed;
2. Providing thrombocytes of a healthy reference living being;
3. Determining the percentage of thrombocytes of step (2) expressing both of P-selectin and phosphatidylserine on their surfaces to obtain a value Mₚᵣₑ;
4. Incubating an aliquot of said blood serum of step (1) with an aliquot of said thrombocytes of step (2);
5. Determining the percentage of thrombocytes after said incubation in step (4) expressing both of P-selectin and phosphatidylserine on their surfaces to obtain a value Mₚₒₛₜ;
6. Diagnosing a predisposition of said living being to develop thrombocytopenia if Mₚₒₛₜ > Mₚᵣₑ.

The present invention also provides for a method for diagnosing a living of suffering from thrombocytopenia, comprising the following steps:
1. Providing blood serum of a living being to be diagnosed;
2. Providing thrombocytes of a healthy reference living being;
3. Determining the percentage of thrombocytes of step (2) expressing both of P-selectin and phosphatidylserine on their surfaces to obtain a value Mₚᵣₑ;
4. Incubating an aliquot of said blood serum of step (1) with an aliquot of said thrombocytes of step (2);
5. Determining the percentage of thrombocytes after said incubation in step (4) expressing both of P-selectin and phosphatidylserine on their surfaces to obtain a value Mₚₒₛₜ;
6. Diagnosing a predisposition of said living being to develop thrombocytopenia if Mₚₒₛₜ > Mₚᵣₑ.

The present inventor has surprisingly realized that a combined presence or expression of P-selectin and phosphatidylserine on the surfaces of thrombocytes is a reliable diagnostic marker of a predisposition of a living being to develop thrombocytopenia and/or of suffering from thrombocytopenia. Especially, the found expression of both P-selectin and phosphatidylserine on the surface of thrombocytes is stimulated by blood serum originating from an individual having a predisposition for or suffering from thrombocytopenia. The latter surprising findings allowed the development of the methods according to the invention.

The art is silent on this phenomenon and it could also not be expected by the skilled artisan.

In contrast to methods known in the art the methods according to the invention are easy to be carried out and generate reliable outcomes. The methods not only allow the diagnosis of a predisposition of a living being to develop thrombocytopenia or to suffer therefrom it also allows a better risk assessment associated with the administration of a vaccine suspected of rarely inducing thrombocytopenia. In case of a diagnosed predisposition the treating physician now has the chance refrain from administering this vaccine and select another vaccine, e.g., an mRNA vaccine, which is not suspected of causing thrombocytopenia or is at least of a lower risk profile.

According to the invention a living being includes any beings, in particular mammals, preferably human beings.

According to the invention, 'blood serum' or simply 'serum' refers to the fluid and solute component of blood which does not play a role in clotting. Serum is the liquid portion of blood obtained as supernatant when a clotted blood sample is centrifuged. It may be defined as blood plasma without fibrinogens. Serum includes all proteins not used in blood clotting; all electrolytes, antibodies, antigens, hormones; and any exogenous substances (e.g., drugs or microorganisms). Serum does not contain white blood cells (leukocytes), red blood cells (erythrocytes), platelets, or clotting factors.

According to the invention 'thrombocytes' or 'platelets' are a component of blood whose function - along with the coagulation factors - is to react to bleeding from blood vessel injury by clumping, thereby initiating a blood clot. Platelets have no cell nucleus; they are fragments of cytoplasm that are derived from the megakaryocytes of the bone marrow, which then enter the circulation. Circulating unactivated platelets are biconvex discoid (lens-shaped) structures, 2-3 µm in greatest diameter.

According to the invention 'P-selectin' or 'CD62', is a type-1 transmembrane protein that in humans is encoded by the SELP gene. P-selectin functions as a cell adhesion molecule (CAM) on the surfaces of activated endothelial cells, which line the inner surface of blood vessels, and activated platelets.

According to the invention 'Phosphatidylserine' (abbreviated Ptd-L-Ser or PS) refers to a phospholipid and is a component of the cell membrane. It plays a key role in cell cycle signaling, specifically in relation to apoptosis.

According to the invention Mₚᵣₑ is a measure for the percentage of thrombocytes of a healthy individual before an incubation with blood serum of a test individual, and 'Mₚₒₛt' is a measure for the percentage of thrombocytes of a healthy individual after the incubation with blood serum of a test individual.

In an embodiment of the invention 'thrombocytopenia' includes thromboses, including cerebral venous sinus thromboses, meaning that the methods according to the invention can also be used to determine a risk of a living being for the development of thromboses or to determine a living being from actually suffering therefrom.

In a variant of the method according to the invention in steps (3) and (5) instead of determining the percentage of thrombocytes expressing both of P-selectin and phosphatidylserine on their surfaces the amount of P-selectin and phosphatidylserine on the surfaces of said thrombocytes is determined.

In an embodiment of the invention said thrombocytopenia is heparin-induced thrombocytopenia (HIT).

By this measure the invention is adapted to the diagnosis of one of the most relevant types of thrombocytopenia for which a reliable and non-complex method to determine a predisposition thereof is currently not available.

In an embodiment of the invention said thrombocytopenia is vaccine-induced immune thrombocytopenia (VITT), preferably by an anti-SARS-CoV-2 vaccine, further preferably an adenovirus-based or adenovirus-associated-based vector vaccine.

By this measure the invention is adapted to a prognostic method allowing the physician a better decision on the risk of the development of a thrombocytopenia when administering vaccines such as ChAdOx1, which are suspected of inducing VITT.

In another embodiment said thrombocytes of step (2) are provided as washed thrombocytes.

This measure has the advantage that the sensitivity of the method is further increased, including the sensitivity of the method to detect pathogenic antibodies.

According to the invention 'washed thrombocytes' are to be understood as including thrombocytes which have had most or preferably essentially all of the plasma, red and white blood cells removed and replaced with saline or another type of preservation solution.

In a yet further embodiment of the invention said thrombocytes of step (2) are provided as platelet rich plasma (PRP).

This measure has the advantage that the time and cost-consuming process of washing the thrombocytes is not required. This also enables the method to be performed by semi-skilled personnel as no experienced laboratory stuff is required.

According to the invention, 'platelet-rich plasma' (PRP), also known as autologous conditioned plasma, is a concentrate of platelet-rich plasma protein from autologous patient whole blood by plasmapheresis using an autotransfusion device or a special tabletop device. The separation principle is based on centrifugal force, by which the individual blood components arrange themselves in layers due to their different specific weights and can then be collected separately (plasmapheresis). The whole blood is separated into the components erythrocytes, platelet-poor plasma (PPP) and platelet-rich plasma (PRP).

In an embodiment of the invention in step 4 platelet factor IV (PF4) is added to said incubation mixture of blood serum and thrombocytes. Alternatively or additionally in step 4 heparin, preferably low molecular weight heparin (LMWH), is added to said incubation mixture of blood serum and thrombocytes.

This measure has the advantage that the sensitivity and/or specificity of the method according to the invention is further increased.

'Platelet factor IV' (PF4) is a small cytokine belonging to the CXC chemokine family that is also known as chemokine (C-X-C motif) ligand 4 (CXCL4). This chemokine is released from alpha-granules of activated platelets during platelet aggregation, and promotes blood coagulation by moderating the effects of heparin-like molecules.

'Heparin' is a naturally occurring polysaccharide that inhibits coagulation, the process that leads to thrombosis. Natural heparin consists of molecular chains of varying lengths, or molecular weights. Chains of varying molecular weights, from 5000 to over 40,000 Daltons, make up polydisperse pharmaceutical-grade heparin. 'Low-molecular-weight heparins' (LMWHs), in contrast, consist of only short chains of polysaccharide. LMWHs are defined as heparin salts having an average molecular weight of less than 8000 Da and for which at least 60% of all chains have a molecular weight less than 8000 Da. These are obtained by various methods of fractionation or depolymerisation of polymeric heparin.

In an embodiment of the invention determining the percentage of said thrombocytes in step 3 and/or step 5 expressing both of P-selectin and phosphatidylserine on their surfaces is carried out via flow cytometry (FC).

This measure has the advantage that a method for determining the newly discovered surface markers is used which is well established and can be easily implemented in daily diagnosis routine.

In another embodiment of the invention in step 6 a predisposition or actual suffering is diagnosed if after said incubation in step (4) ≥ 10% of said thrombocytes have expressed both of P-selectin and phosphatidylserine on their surfaces.

This measure has the advantage that a particular threshold is provided which has turned out as resulting in a reliable and simplified diagnosis.

While in embodiments of the invention the percentage of thrombocytes expressing both of P-selectin and phosphatidylserine on their surfaces can also be ≥ 1%, ≥ 2%, ≥ 3%, ≥ 4%, ≥ 5%, ≥ 6%, ≥ 7%, ≥ 8%, ≥ 9%, it is preferred if the percentage is ≥ 10%. This includes, according to the invention, a percentage of ≥ 15%,≥ 20%,≥ 25%,≥ 30%,≥ 35%,≥ 40%,≥ 45%,≥ 50%,≥ 55%,≥ 60%,≥ 65%,≥ 70%,≥ 75%,≥ 80%,≥ 85%,≥ 90%,≥ 95% and ≥ 100%.

Another subject-matter of the invention relates to the use of a combined presence of P-selectin and phosphatidylserine on the surface of thrombocytes as a diagnostic marker of a predisposition of a living being to develop thrombocytopenia and/or of suffering from thrombocytopenia, preferably heparin-induced thrombocytopenia (HIT) and/or vaccine-induced immune thrombocytopenia (VITT).

The features, characteristics, advantages and embodiments of the methods according to the invention apply to the use according to the invention *mutatis mutandis.*

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

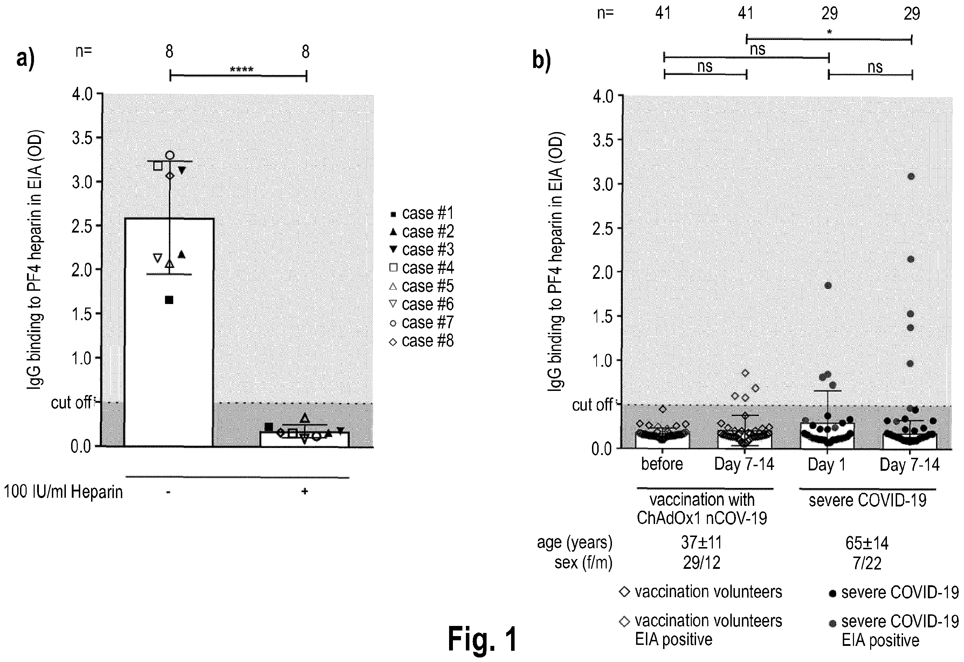

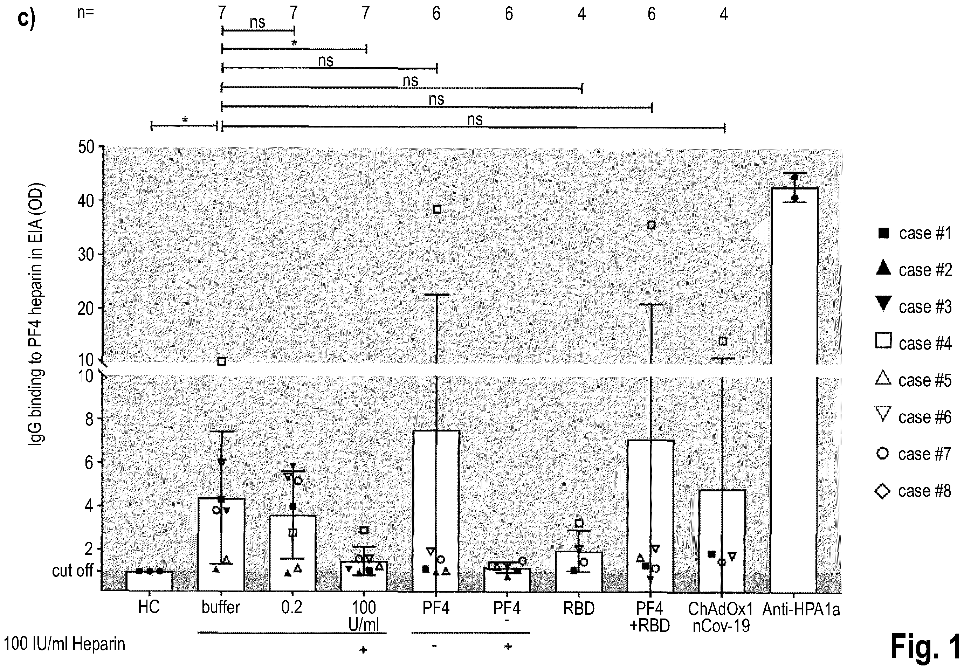

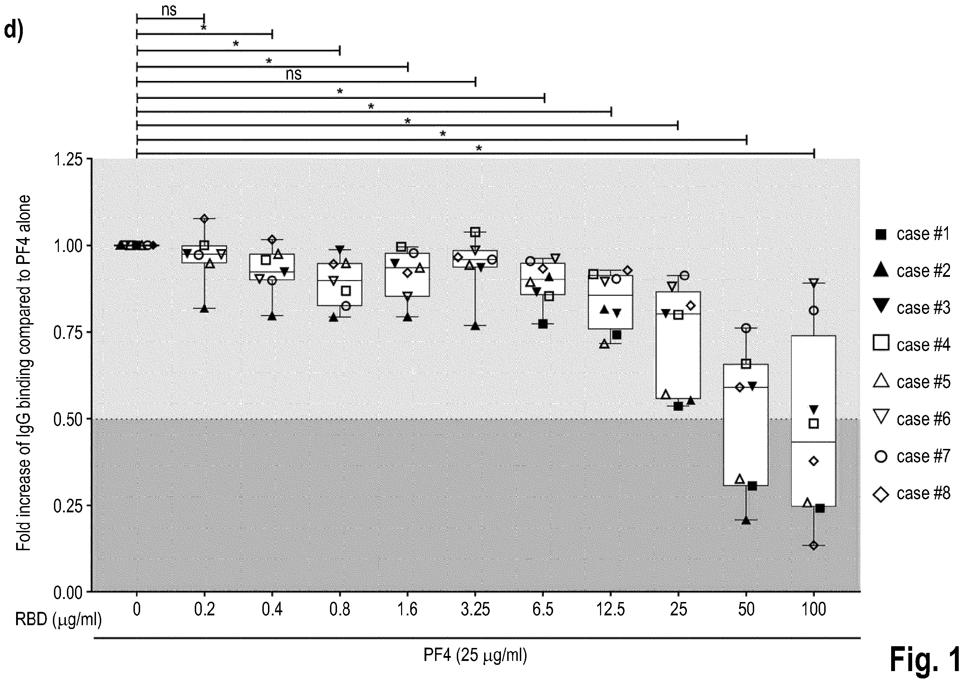

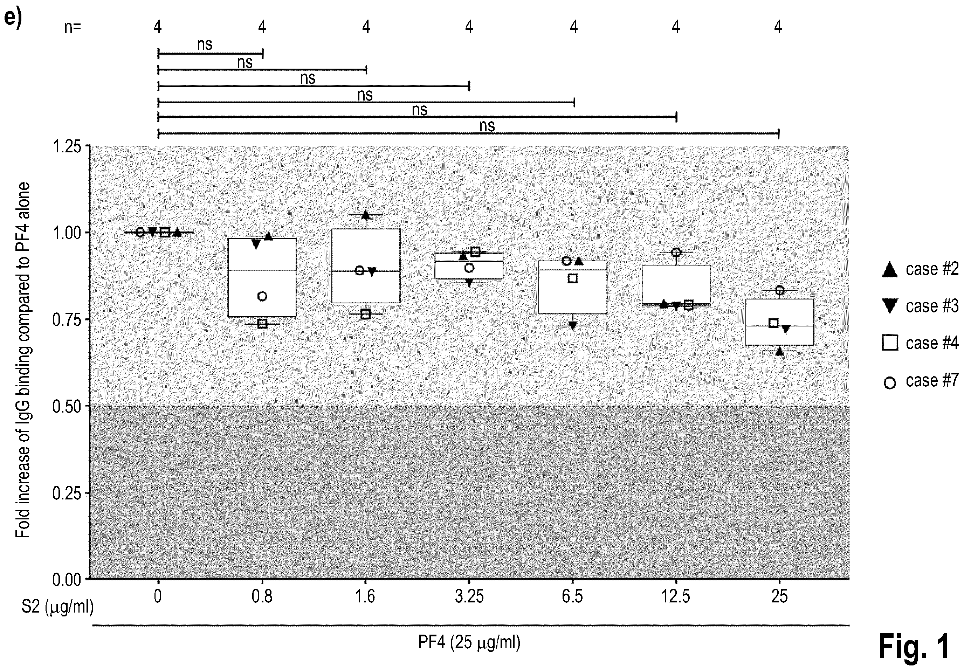

- Fig. 1:: Antibody-mediated platelet activation and generation of procoagulant platelets. Results of the platelet activation assay (HIPA) with modifications in the VITT-patients. Each dot represents the median of four different donors. All VITT patients presented platelet activation with buffer alone, which was significantly increased by PF4 heparin but inhibited with high dose of heparin (Panel A). Procoagulant platelets (CD62P/Phosphatidylserine (PS) positive) cells in different settings were analyzed via Annexin V-FITC and CD62p-APC antibody staining. Where indicated, PLTs were treated with PF4, 0.2U/ml and 100U/ml heparin, RBD and ChAdOx1 nCoV-19A (Panel B). Results of HIPA at different titrations of sera from VITT patients. Note that diluted sera (from 1:64) activated platelets only in the presence e of PF4 (Panel C). Effect of sera from VITT patients at different titrations on the development of procoagulant platelet. Note that diluted sera (from 1:8) activated platelets only in the presence e of PF4 (Panel D). Data are presented as mean ± standard deviation (SD) of the measured fold increase compared to control, not significant, *p<0.05, **p<0.01, ***p<0.001 and ****p<0.0001. The number of sera tested is reported in each graphic. Dot lines represent the cutoffs determined testing sera from healthy donor.

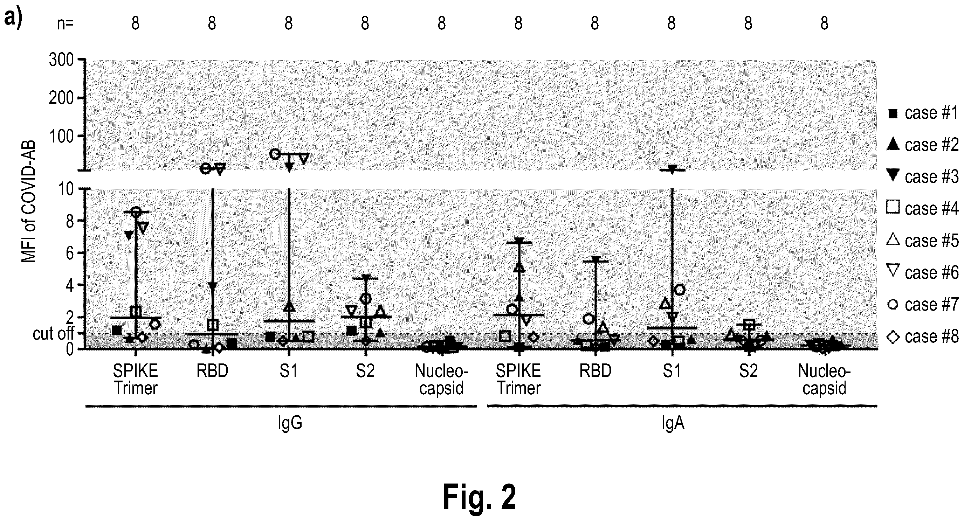

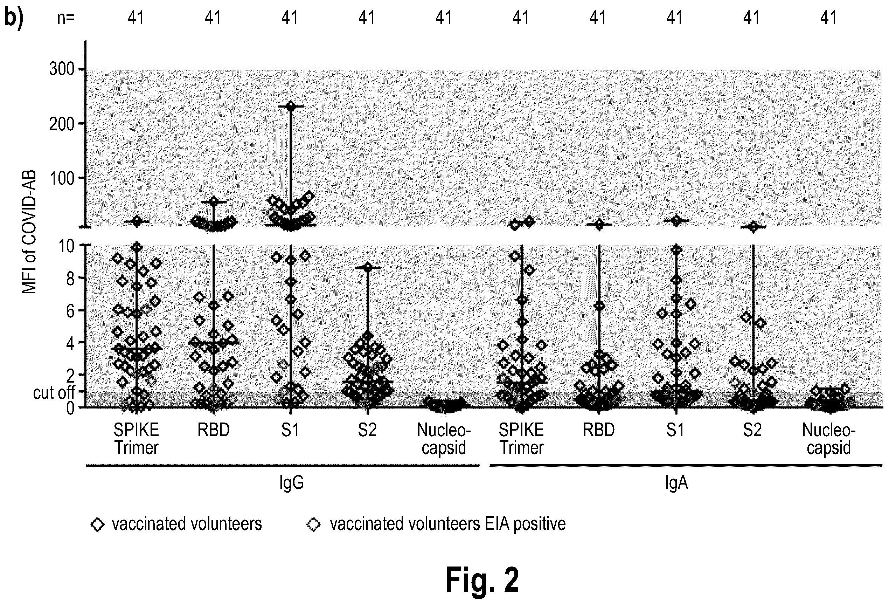

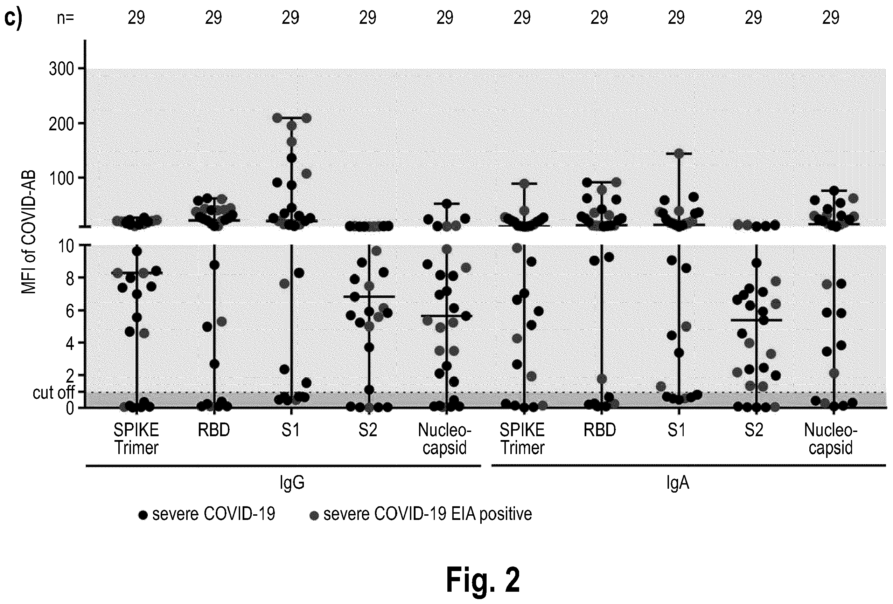

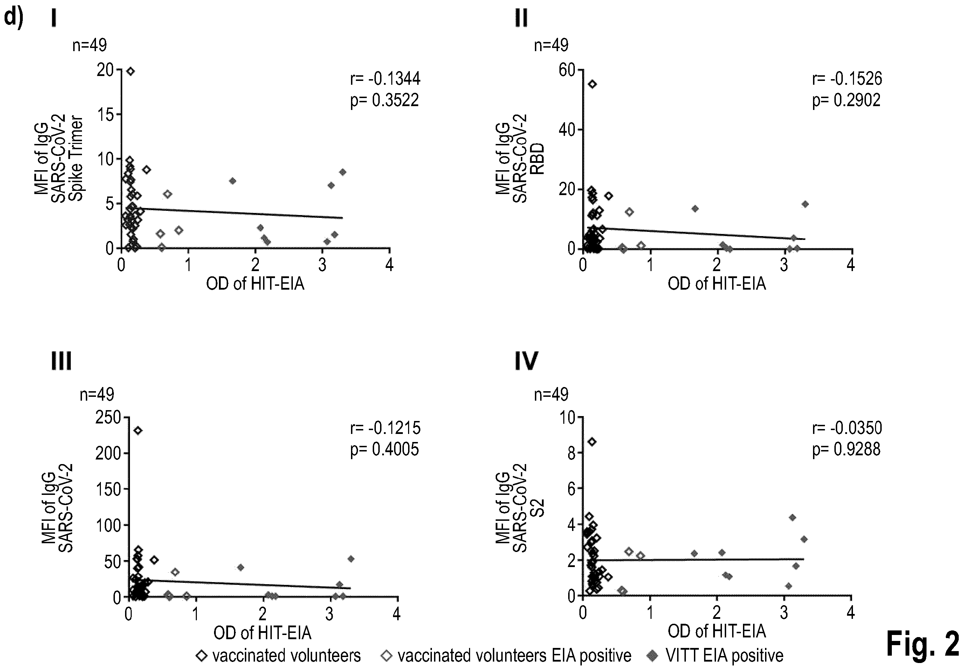

- Fig. 2:: Serum anti-COVID IgG and IgA antibody levels and correlation with HIT-EIA. This figure shows the MFI of anti-COVID IgG and IgA antibodies values for Spike Trimer, RBD, S1, S2 and nucleocapsid quantified by using a bead-based Luminex assay in VITT patients (Panel A); vaccinated volunteers (Panel B) and COVID-19 patients (Panel C). Anti-PF4 anti-body levels did not correlate with anti-COVID antibodies in VITT patients and vaccinated volunteers (Panel D). Each symbol shows an individual subject and numbers of tested subjects is indicated in the graphic.

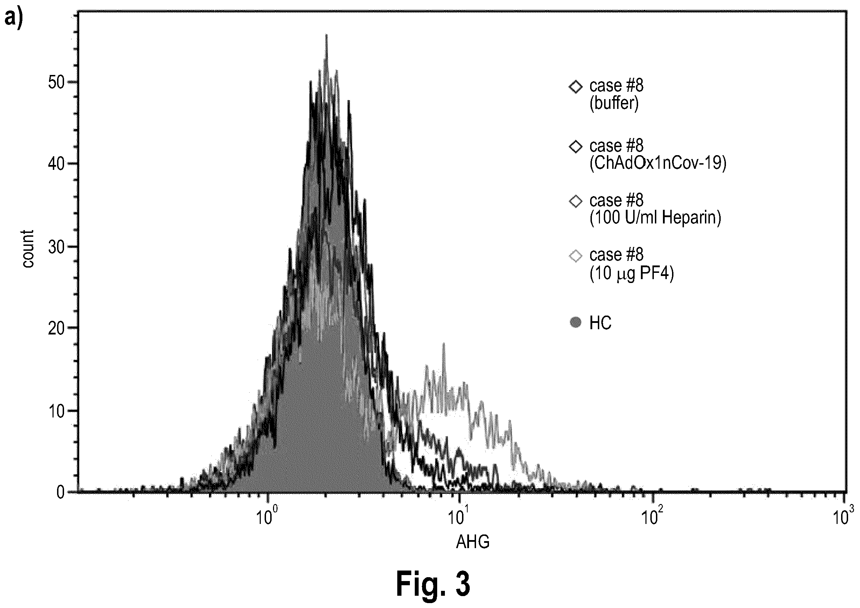

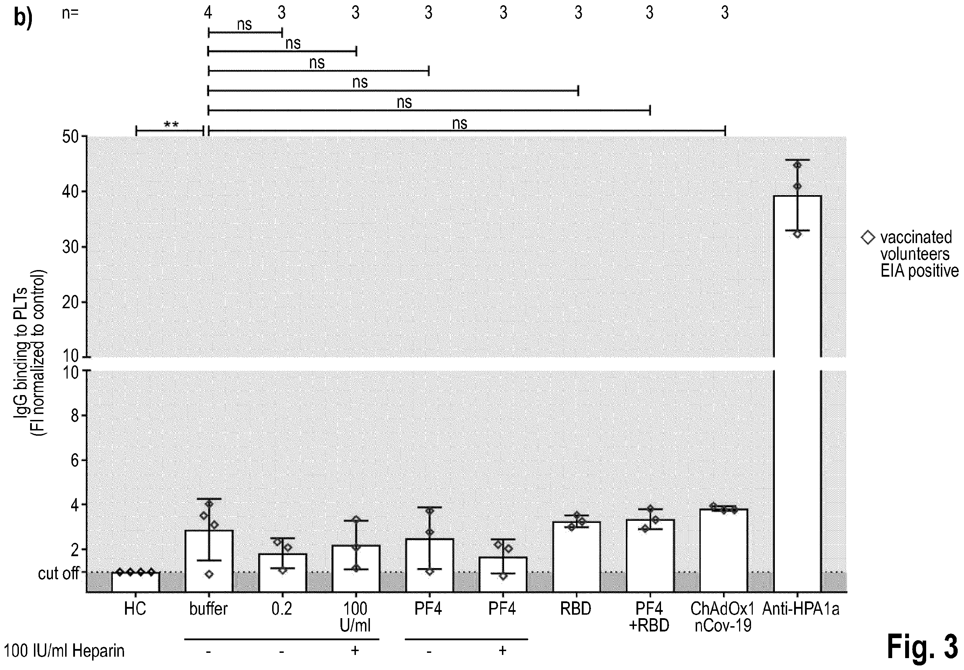

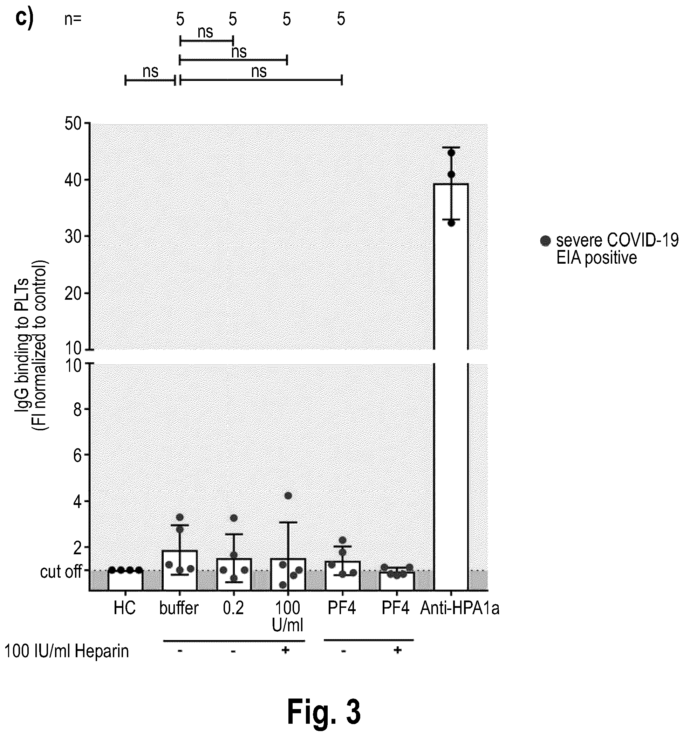

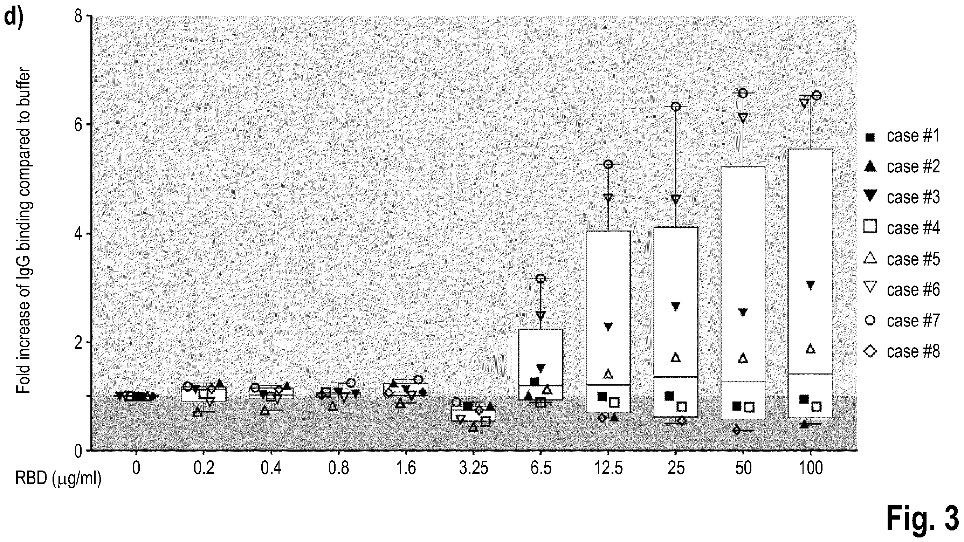

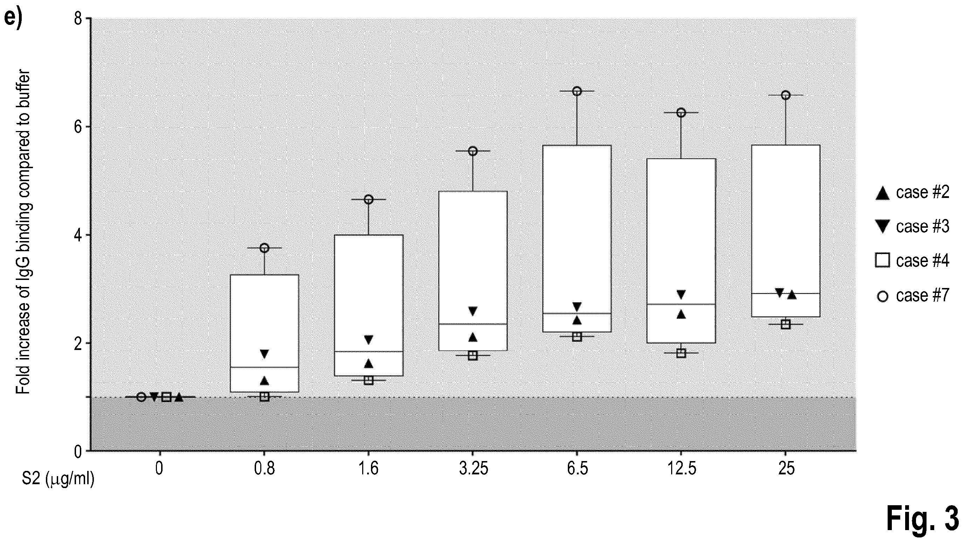

- Fig. 3:: IgG binding to healthy PLTs. Panel A shows a representative flow cytometry histogram of AHG binding. The AHG-binding was measured in presence of buffer and high concentration of heparin (100 IU/ml heparin) after incubation of platelets from healthy donors (HC) with serum of VITT patients. The other panels show IgG binding to healthy PLTs (assessed by flow cytometry and expressed as fold increase normalized to controls) after incubation with sera from vaccinated volunteers (Panel B) and COVID-19 patients (Panel C). Where indicated, PLTs were treated with PF4, 0.2U/mL and 100U/mL Heparin, RBD and the vaccine ChA-dOx1 nCoV-19. (Abbreviation: ns: not significant, and **p<0.01). IgG binding to SARS-CoV-2 RBD was tested for VITT patients and assessed by EIA (expressed as fold increase to buffer controls). An increased tendency of IgG binding to RBD was observed at increasing concentrations of RBD (Panel D). IgG binding to SARS-CoV-2 S2 was not observed in VITT patients (Panel E). Each symbol represents individual subject and the number subjects tested is reported in each graphic.

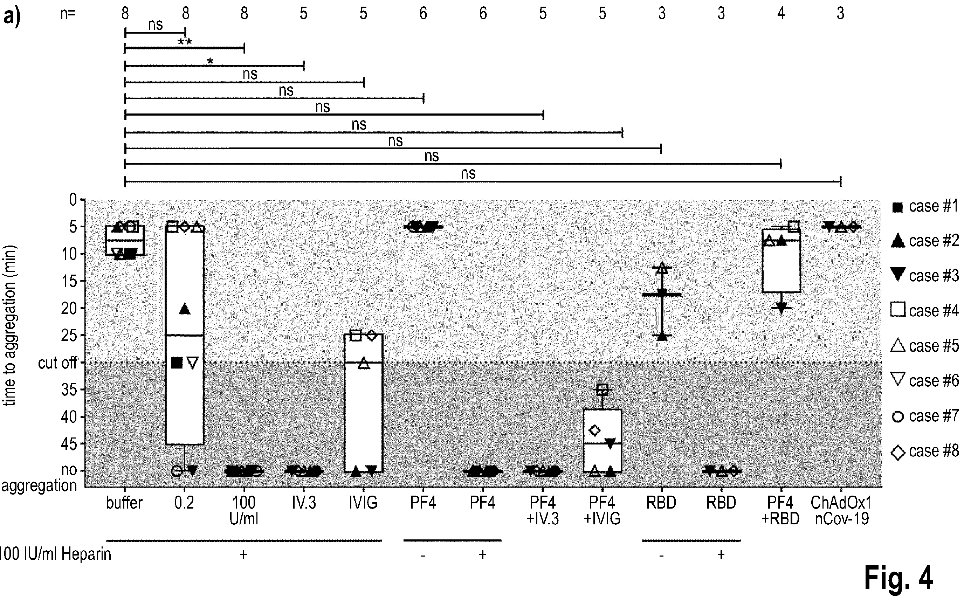

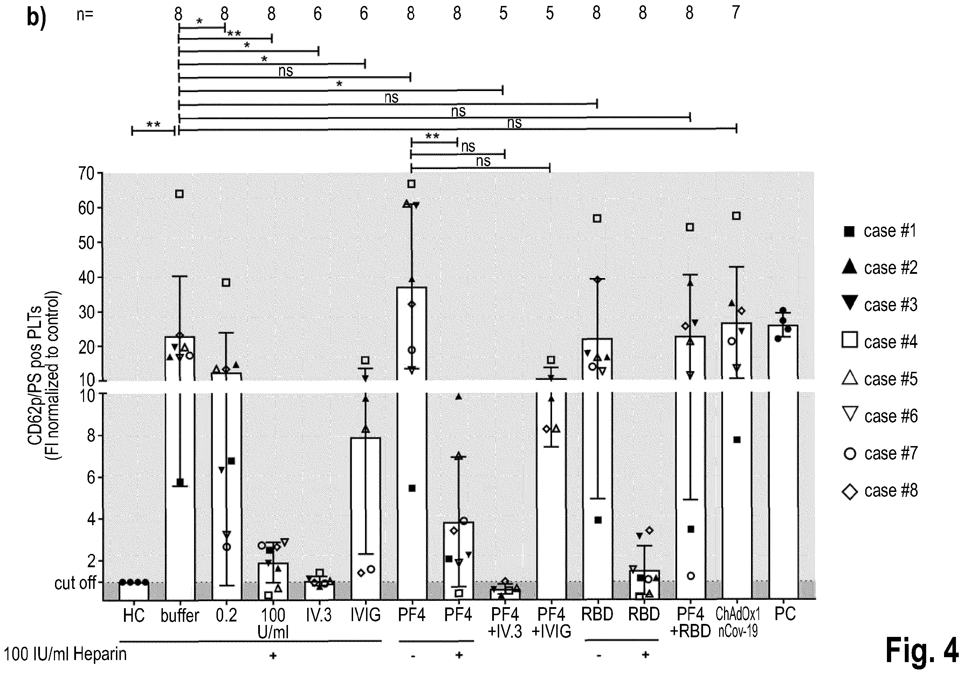

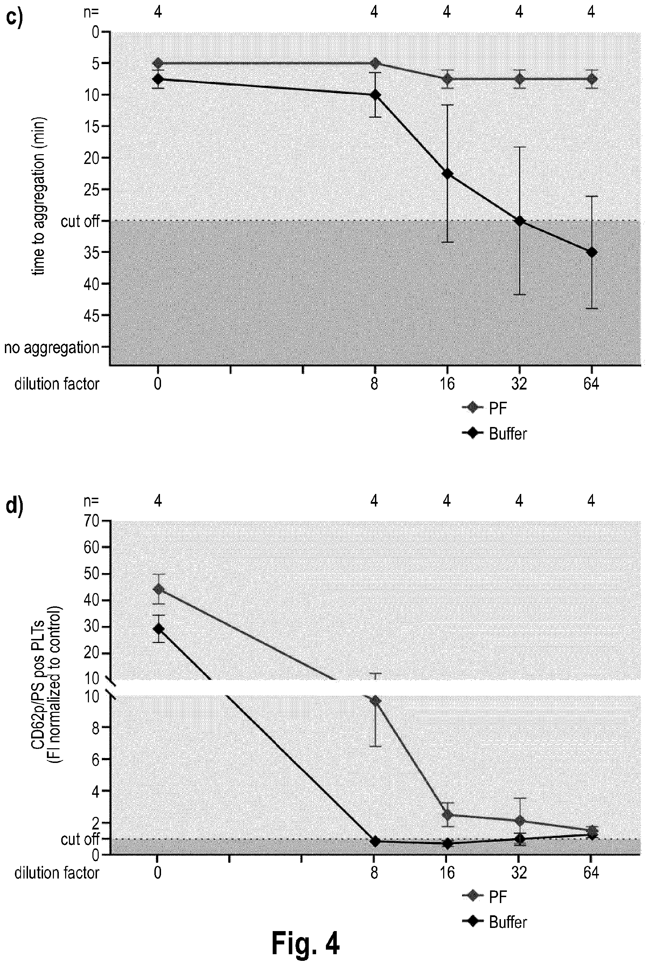

- Fig. 4:: Antibody-mediated platelet activation and generation of procoagulant platelets. Results of the platelet activation assay (HIPA) with modifications in the VITT-patients. Each dot represents the median of four different donors. All VITT patients presented platelet activation with buffer alone, which was significantly increased by PF4 heparin but inhibited with high dose of heparin (Panel A). Procoagulant platelets (CD62P/Phosphatidylserine (PS) positive) cells in different settings were analyzed via Annexin V-FITC and CD62p-APC antibody staining. Where indicated, PLTs were treated with PF4, 0.2U/ml and 100U/ml heparin, RBD and ChAdOx1 nCoV-19A (Panel B). Results of HIPA at different titrations of sera from VITT patients. Note that diluted sera (from 1:64) activated platelets only in the presence e of PF4 (Panel C). Effect of sera from VITT patients at different titrations on the development of procoagulant platelet. Note that diluted sera (from 1:8) activated platelets only in the presence e of PF4 (Panel D). Data are presented as mean ± standard deviation (SD) of the measured fold increase compared to control, not significant, *p<0.05, **p<0.01, ***p<0.001 and ****p<0.0001. The number of sera tested is reported in each graphic. Dot lines represent the cutoffs determined testing sera from healthy donor.

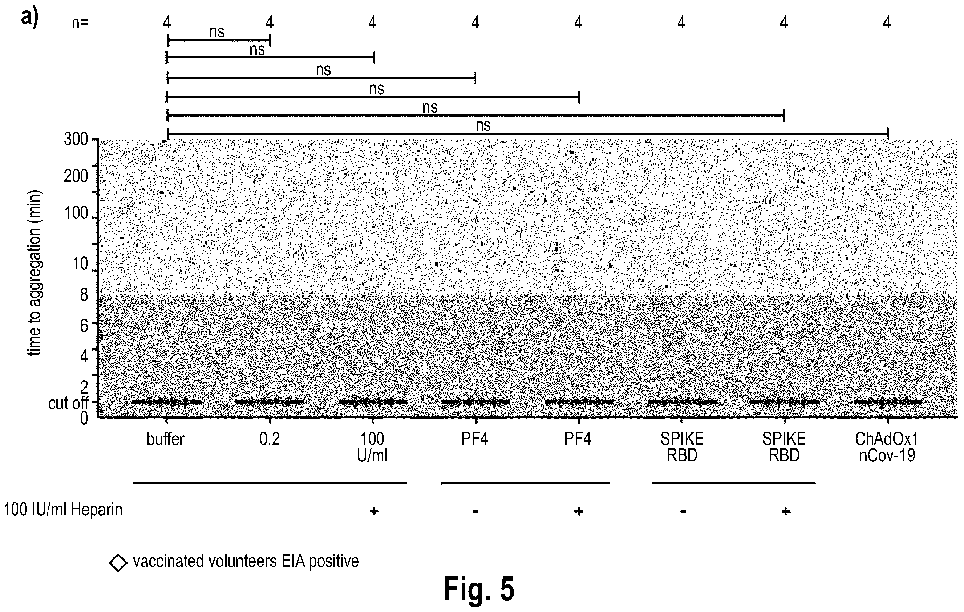

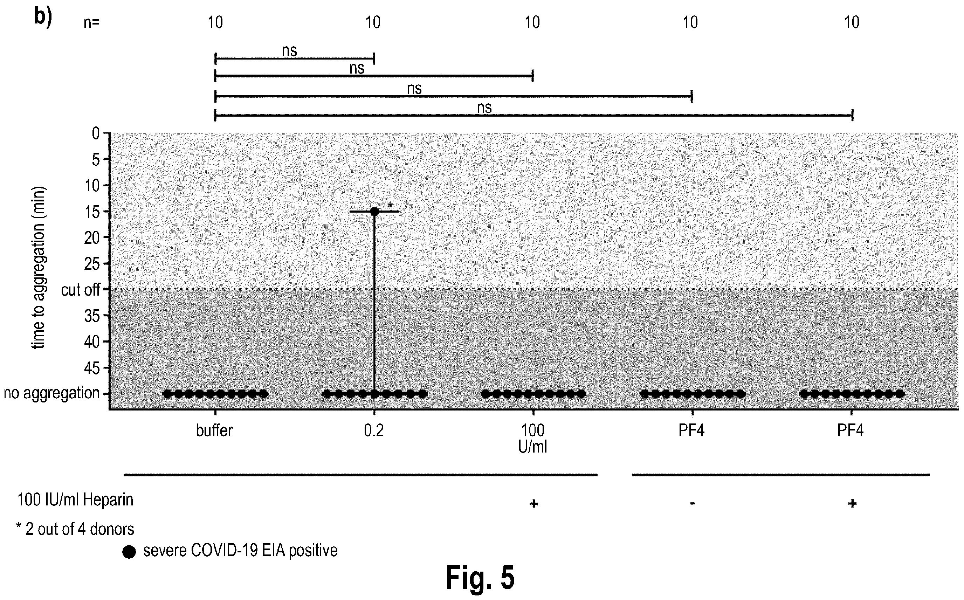

- Fig. 5:: Heparin-induced platelet activation assay (HIPA). This figure shows the results of the platelet activation assay (HIPA) with modifications in presence or absence of PF4, 0.2U/ml and 100U/ml Heparin, RBD and vaccine ChAdOx1 nCoV-19. None of the subjects showed any platelet activation under any conditions except one in presence of Spike RBD in vaccinated controls (Panel A). Similar results were seen for COVID-19 patients except one patient who showed enhanced activation in 2 out of 4 donors in the presence of 0.2U/ml heparin (*p<0.05, Panel B). Each symbol represents individual subject and the number of subjects tested is reported in each graphic.

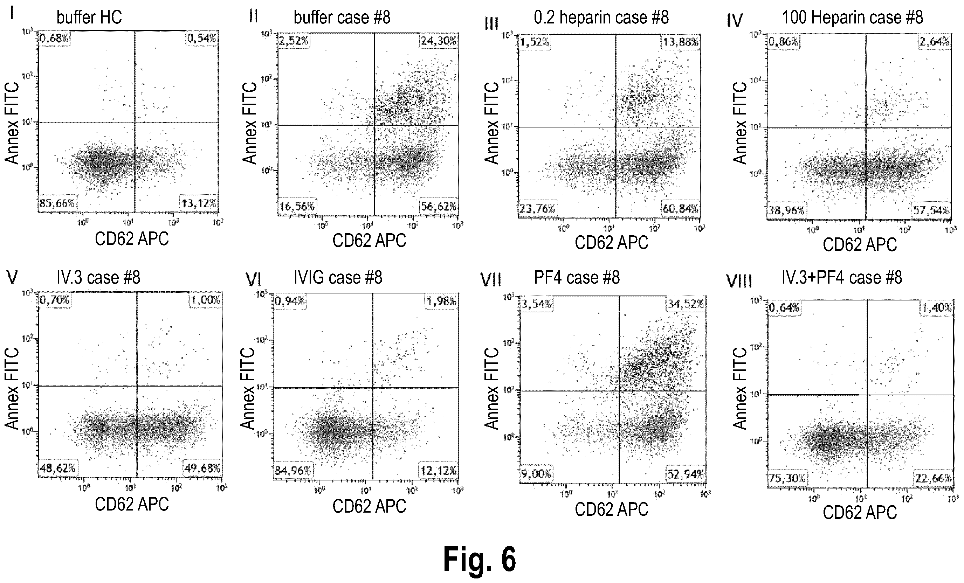

- Fig. 6:: Representative dot plot histograms of procoagulant platelets. Washed platelets (PLTs) were incubated with serum from VITT patients. I-VIII show representative dot plots in presence of [I] buffer HC, [II] buffer case #8, [III] 0.2 heparin case #8, [IV] 100 heparin case #8, [V] IV.3 case #8, [VI] IVIG case #8, [VII] PF4 case #8 and [VIII] VI.3 + PF4 case #8.

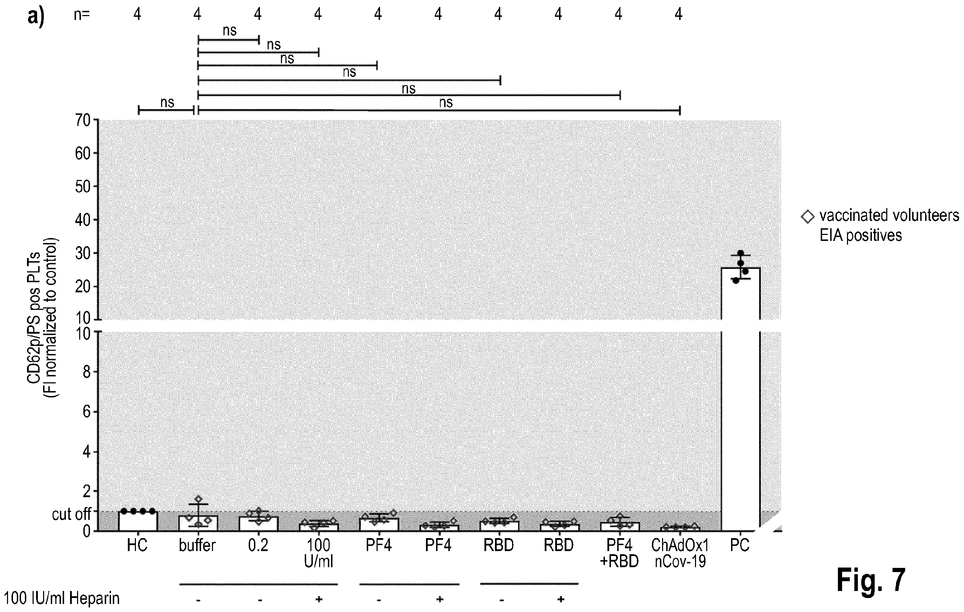

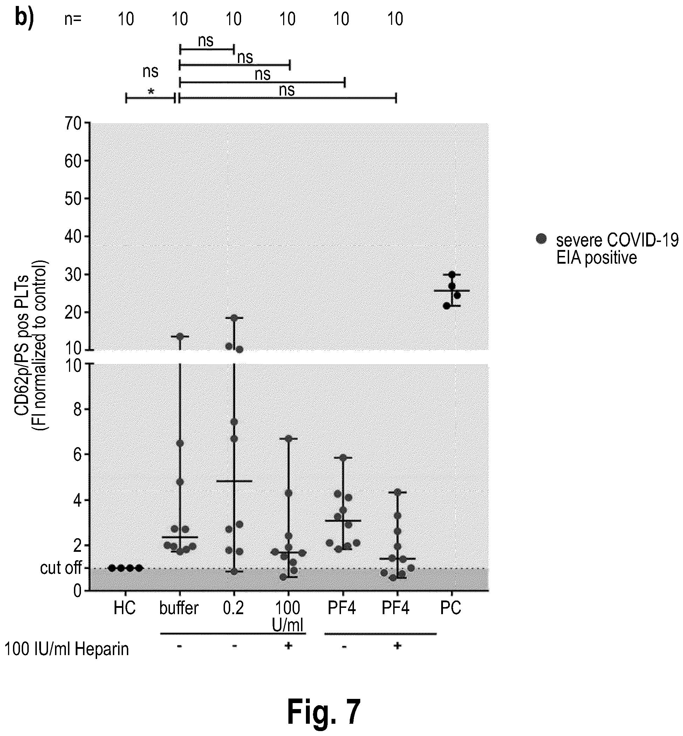

- Fig. 7:: Procoagulant platelets in vaccinated volunteers und COVID-19 patients. Procoagulant platelets (CD62P/Phosphatidylserine (PS) positive cells in different settings with sera from vaccinated volunteers (Panel A) and COVID-19 patients with anti PF4 antibodies (Panel B). Data are presented as mean ± standard deviation (SD) of the measured fold increase compared to control, not significant, and *p<0.05. Dot lines represent the cutoffs determined testing sera from healthy donors as mean of fold increase (FI).The number of sera tested is reported in each graphic.

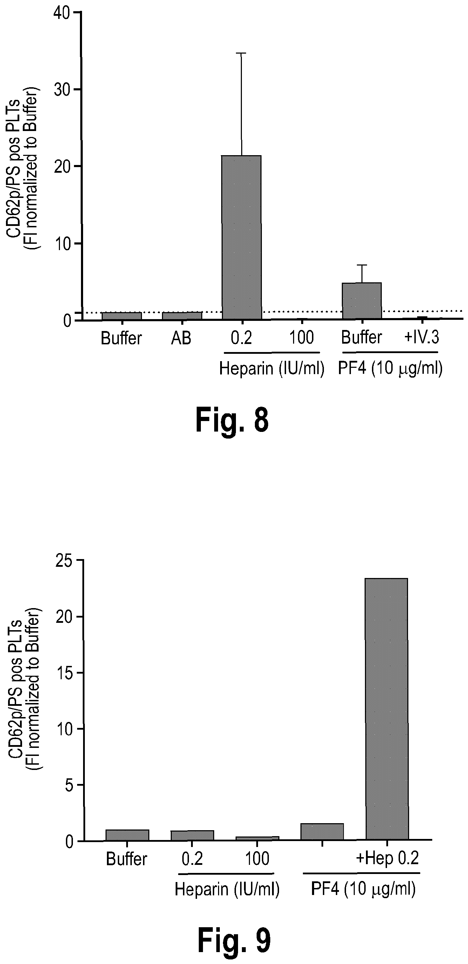

- Fig. 8:: Procoagulant platelets (CD62P/Phosphatidylserine (PS) positive cells in different settings after incubation of sera with washed PLTs. PLTs were analyzed via Annexin V-FITC and CD62p-APC antibody staining. Where indicated, PLTs were treated with PF4, 0.2U/ml and 100U/ml heparin, and monoclonal antibody IV.3. Data are presented as mean ± standard error mean (SEM) of the measured fold increase compared to buffer control.
- Fig. 9:: Procoagulant platelets (CD62P/Phosphatidylserine (PS) positive cells in different settings after incubation of HIPA- sera with PRP. PLTs were analyzed via Annexin V-FITC and CD62p-APC antibody staining. Where indicated, PLTs were treated with PF4, 0.2U/ml and 100U/ml heparin. Data are presented as mean ± standard error mean (SEM) of the measured fold increase compared to buffer control.

### EXAMPLES

### 1. Introduction

COVID-19 infection has resulted in considerable morbidity and mortality in the last 15 months. Within an exceptionally short time, several SARS-CoV-2 vaccines have been licensed and used worldwide. Safety signals have been, however, noted. Center for disease control and prevention (CDC) in the US reported in the beginning of 26 March 2021 cases of venous thromboembolism, 20 cases of thrombosis and 41 ischemic strokes in individuals vaccinated with mRNA vaccines in the US. More than 200 cases with thrombosis among 34 million persons vaccinated with ChAdOx1 nCoV-19 have been reported to European database of suspected adverse reactions, EudraVigilance. After the investigation of reported cases, European Medical Association (EMA) found a link between ChAdOx1 nCoV-19 and unusual thrombotic events and concomitant thrombocytopenia. Although WHO and EMA concluded that the benefit of vaccination with ChAdOx1 nCoV-19 outweighs the risks associated with thrombosis and thrombocytopenia, several countries instituted restrictions on the use of ChAdOx1 nCoV-19. The unusual clinical constellation of cerebral venous sinus thrombosis (CVST) and thrombocytopenia is called vaccine-induced immune thrombotic thrombocytopenia (VITT). The inventors studied 8 cases with thrombocytopenia and primarily with suspected CVST but also other thromboembolic complications to better understand the pathophysiology of the VITT. In this study, the inventors identified antibody-mediated procoagulant platelets as a novel mechanism associated with VITT.

### 2. Methods

### Study cohort and evaluation of the clinical data

Blood samples were collected to analyze the coagulation parameters and to exclude heparin-induced thrombocytopenia (HIT). Blood samples from non-vaccinated healthy blood donors (n=24) served as health controls (17 females, mean age 36.1±13.7 years). Blood samples were also collected from the coworkers of Blood Donation Center Tübingen and University Hospital Ulm before and after the first vaccination with ChAdOx1 nCoV-19 to serve as vaccinated controls (n=41, 29 females, mean age 37.3 ± 10.9 years). None of the coworkers developed hematological abnormalities. All subjects in this study received ChAdOx1 nCoV-19 vaccine (Vaxzevria, Astra-Zeneca, London, UK). In addition, sera from 29 COVID-19 patients who had serial HIT EIA measurements during hospitalization were also included in the study (7 females, mean age 65.3 ± 14.1 years). Clinical data from 21 of these ICU COVID-19 patients were reported in a previous study.

### Bead-based multiplex assay for detection of COVID-19 antibodies

COVID-19 antibodies were measured with a multiplex assay (NMI, Reut-lingen, Germany) with the FLEXMAP 3D^{®} system (Luminex Corporation, Austin, USA). Tests were performed at the Blood Donation Center of Tübingen.4 Bound antibodies were detected by a single measurement with the Luminex FLEXMAP 3D^{®} and the Luminex xPONENT Software 4.3 (settings: 50 events, Gate: 7,500-15,000, Reporter Gain: Standard PMT).

### Testing for anti-PF4/heparin antibodies

A commercially available IgG-Enzyme Immune Assay (EIA) was used in accordance to manufacturer's instructions (Hyphen Biomed, Neuville-sur-Oise, France). Per manufacturer's recommendations, a sample was considered reactive if the optical density (OD) was ≥0.500. The ability of sera to activate platelets was tested using the functional assay heparin induced platelet aggregation assay (HIPA) as previously described.

### Serological characterization of PF4-antibodies

Antibody binding to PF4 and Receptor Binding Domain of Spike Protein (Spike-RBD) was analyzed using an In-House EIA. PF4 (25 µg/mL, ChromaTec, Greifswald, Germany) and SPIKE-RBD domain (0-100µg/mL) were immobilized onto microtiter plates (Nunc MaxiSorp, Thermo Fisher Scientific Inc., Waltham MA, USA) at different concentrations.

### Assessment of antibody-mediated procoagulant platelets

To exclude unspecific effects like the activation of platelets via complement or non-specific immune complexes, all sera were heat-inactivated (56°C for 30 min*), followed by a sharp centrifugation step at 5,000g. The supernatant was collected. All experiments involving patients' sera were performed after incubation of 5 µL serum with 25 µL washed platelets (7.5x10⁶) for 1.5 h under rotating conditions at RT. When indicated, cell susspenions were preincubated with PF4 (25 µg/ml), Spike protein (0-100 µg/mL) or vaccine (1:75, V:V). Afterwards, samples were washed once (7 min, 650g, RT, without brake) and gently resuspended in 75 µL of phosphate-buffered saline (PBS, Biochrom, Berlin, Germany). Platelets were then stained with Annexin V-FITC and CD62-APC (Immunotools, Friesoythe Germany) and directly analyzed by flow cytometry (FC). As positive control, washed platelets were incubated with ionomycin (5µM, 15 min at RT) and TRAP-6 (10 µM, 30 min at RT). Test results were determined as fold increase of the percentage of double PS/CD62p positive events in platelets upon incubation with patients' sera compared to cells incubated with healthy donors tested in parallel.

### Assessment of antibody-mediated procoagulant platelets using washed platelets

Prior to usage, all sera were heat-inactivated at 56°C for 30 min, followed by a sharp centrifugation step at 5,000g. The supernatant was collected in a fresh tube. For determination of procoagulant platelets, 5 µL serum was incubated with 25 µL washed platelets (7.5x106) for 1 h under rotating conditions at RT. Where indicated, cell susspenions were preincubated with PF4 (10 µg/ml) and Heparin (0.2 or 100 IU/ml). Afterwards, samples were washed once (7 min, 650g, RT, without brake) and gently resuspended in 75 µL of phosphate-buffered saline (PBS, Biochrom, Berlin, Germany). Platelets were then stained with Annexin V-FITC and CD62-APC (Immunotools, Friesoythe Germany) and directly analyzed by flow cytometry (FC). Test results were determined as fold increase of the percentage of double PS/CD62p positive events in platelets upon incubation with patients' sera compared to the baseline.

### Assessment of procoagulant platelets in platelet rich plasma (PRP)

For determination of procoagulant platelets in PRP, sera were prepared as described above. For PRP preparation, venous blood from healthy individuals was withdrawn into vacutainers containing sodium citrate 0.105 M (3.2%) (BD, Plymouth, UK) and allowed to rest for 20 min at RT. PRP was prepared by centrifugation (20 min, 120g, RT) and adjusted with autologous platelet poor plasma (PPP, [10 min, 2000g, RT]) to a PLT count of 300x10⁶/ml. 5 µL serum was incubated with 37.5 µL PRP (11.25x10⁶ cells), filled up to 50 µl with PBS, and incubated for 1h at RT under rotating conditions. Where indicated, PRP was preincubated with PF4 (10 µg/ml) and Heparin (0.2 or 100 IU/ml). Afterwards, samples were processed as described for washed PLTs and analysed by flow cytometry (FC). Test results were determined as fold increase of the percentage of double PS/CD62p positive events in platelets upon incubation with patients' sera compared to the baseline.

### Ethics Statement

The study was conducted in accordance with the declaration of Helsinki. Written informed consent was obtained from all volunteers, VITT patients or their relatives prior to any study-related procedure. All tests were performed with leftover material from routine testing. The study protocol was approved by the Institutional Review Board of the University of Tuebingen (236/2021BO1). Collection and analysis of sera from ChAdOx1 nCoV-19 vaccinated individuals were approved by Ethics Committee of Ulm University (99/21).

### Statistical analyses

The statistical analysis was performed using GraphPad Prism, Version 7.0 (GraphPad, La Jolla, USA). Since potential daily variations in FC measurements might result in bias in data analysis, test results were normalized to two healthy donors tested in parallel at the same time point (raw data are available in the supplemental data). Data in the text are presented as median (range), mean ± standard deviation (SD) or n (%).

### 3. Results

### IgG binding profile of sera from VITT

High titer PF4/heparin antibodies were detected in all sera (8/8 100%) using the IgG PF4/heparin EIA. Interestingly, binding of all sera was inhibited in the presence of high concentration of heparin (mean optical density [OD] of IgG antibodies against PF4/heparin complexes: 2.591±0.642 vs. 0.176±0.073, respectively, p < 0.0001, Figure 1A). No correlation was found between the PF4/heparin antibodies and the detected COVID-19 antibodies in VITT patients and in vaccinated controls (Figure 2 A-D [I-IV]). Among non-vaccinated controls only one subject (4%) had a PF4/heparin antibody in EIA (Data not shown).

The inventors next investigated the PF4-seroconversion after vaccination with ChAdOx1 nCoV-19, as well as during severe SARS-CoV-2 infection (Figure 1 B). The inventors found that 4 out of 41 (9.8%) vaccinated healthy individuals and 4 out of 41 (9.8%) patients with severe COVID-19 seroconverted with IgG antibodies against PF4/heparin complexes within 14 days (Figure 1B). Next, the inventors tested IgG binding to platelets by FC. An increase in IgG binding to test platelets was observed (Fold increase in mean fluorescence intensity compared to healthy controls [FI of MFI]: 4.39±1.15 vs. 1±1.10, p value 0.026, Figure 1C, Figure 3A). IgG binding to platelets was inhibited by heparin at high concentrations (FI of MFI IgG binding: 1.51±0.66, p value 0.016), but not at low concentrations (FI of MFI of IgG binding: 3.60±2.01, p value 0.688). Only one serum showed increased binding to platelets in the presence of PF4 and the vaccine ChAdOx1 nCoV-19 (case #4, Figure 1C). Spike-RBD did not induce a significant change in IgG binding in VITT patients (Figure 1C). Similar results were observed when S2 protein is added (Figure 1E). IgG binding was also observed when sera from ChAdOx1 nCoV-19 vaccinated volunteers with IgG PF4 antibodies were tested (Figure 3B). However, severe COVID-19 patients with IgG PF4 antibodies showed no increase in IgG binding (Figure 3C).

### The impact of Spike-RBD on the binding of anti-PF4 antibodies

Compared to healthy controls, sera from VITT patients showed a strong binding to PF4 in the in-house EIA (OD IgG antibodies against PF4: 1.03±0.04 vs. 0.110±0.002, respectively, p value <0.0001, Figure 1C). On the other hand, sera from VITT patients showed slight but not significant binding to Spike-RBD (Figure 3D). Most importantly, in the presence of PF4 the IgG binding was reduced when the concentration of RBD is increased above 6.5 µg/mL (Figure 1D). However, sera from VITT patients did not show a significant binding to S2 protein with and without PF4 (Figure 1E, Figure 3E).

### Platelet activation in the HIPA assay

To investigate the ability of patients sera to activate platelets, the HIPA assay was used with several modifications. Sera were incubated with washed platelets in the presence of I) buffer, II) 0.2 IU/mL LMWH, III) 100 IU/mL UFH, IV) an Fc gamma receptor IIa (FcγRIIA)-blocking monoclonal antibody (mAb IV.3), VI) 30mg/mL IVIG, VII) 25 µg/mL PF4, VIII) 50 µg/mL Spike-RBD, IX) PF4/Spike-RBD complexes, X) PF4+RBD or XI) ChAdOx1 nCoV-19 (XII). Conditions with PF4 and RBD were also repeated in the presence of high concentration of heparin (100 IU/mL UFH). We observed platelet activation in presence of buffer in 8/8 VITT patients (Median time to platelet aggregation: 5, 5-10 minutes (min), Figure 4A), but not in sera from vaccinated individuals with anti PF4 antibodies, who did not develop any clinical sign of thromboembolic complications, without side effects (Figure 5A). Moreover, only one of the sera from patients with severe COVID-19 who were tested positive in PF4/heparin EIA showed platelet activation in the HIPA assay. (Figure 5B). Interestingly, the reaction was weaker in presence of low molecular weight heparin (Median time to aggregate: 5 min, 5-10 min (no aggregation) vs. 30 min, 5->45min, Figure 4A). All reactions were inhibited by high dose of heparin (p value 0.008, Figure 4A). In presence of PF4, sera from VITT patients showed strong platelet activation (Median time to aggregate: 5 min, 5-5 min, Figure 4A). Most importantly, platelet activation was completely inhibited by the mAb IV.3 that blocks the FcyRlla and by high doses of IgG (>45 min, no aggregation, Figure 4A). No significant change was found when PF4/RBD-complexes were added. Antibody-mediated platelet activation was inhibited when low molecular weight heparin (LMWH) was added at low concentrations by testing three sera. When sera from VITT patients were diluted, specific binding was observed to PF4 while no reaction in the buffer was found (Figure 4C).

### Sera of VITT patients induce PF4-dependent procoagulant phenotype

To explore the mechanism of coagulation dysregulation in VITT, sera were incubated with washed platelets from healthy donors in the presence of buffer, heparin, mAb IV.3, IVIG, PF4, PF4+IVIG, PF4+RBD, the Spike-RBD protein or the vaccine ChAdOx1 nCoV-19. FC analyses revealed that sera from VITT patients induce remarkable changes in the distribution of CD62p/PS positivity (FI CD62p/PS positive PLTs: 22.94±6.14 vs. 0.90±0.63, respectively, p=0.009, Figure 4B, Figure 6). In contrast, the platelet population was almost non-affected after incubation with sera from vaccinated controls (Figure 7A). Interestingly, the generation of procoagulant platelets was reduced by 0.2 IU/mL LMWH (FI CD62p/PS positive PLTs: 13.32±11.50, p=0.016, Figure 4B) and completely inhibited by high concentration of unfractionated heparin (UFH) in VITT patients (FI CD62p/PS positive PLTs: 1.92±0.96, p= 0.008, Figure 4B, Figure 6). These reactions were inhibited by the FcγRIIA blocking with mAb IV.3 as well as by high concentrations of IgG (FI CD62p/PS positive PLTs: 1.04±0.22, p= 0.031 and FI CD62p/PS positive PLTs: 7.88±5.56, p= 0.031, respectively, Figure 4B). No significant increase of procoagulant platelets was also observed in presence of PF4 (FI CD62p/PS positive PLTs: 37.07±23.73, p=0.078) and in the presence of Spike-RBD alone (FI CD62p/PS positive PLTs: 22.02±17.09, p=0.195). While increased generation of procoagulant platelets was observed after incubation of sera from severe COVID-19 patients, no significant change was observed when sera from vaccinated individuals with anti-PF4 antibodies were tested (Figure 7A-B).

To identify the target antigen of the platelet activating antibodies, the HIPA and FACS testing were repeated at different titrations of sera from VITT patients. Interestingly, diluted sera (from 1:64) were able to activate platelets and induce procoagulant phenotype only in the presence e of PF4 (Figure 4 C and D respectively).

### Procoagulant Platelets in Heparin-Induced Thrombocytopenia

Sera from patients were incubated with washed platelets (Fig. 8) or PRP (Fig. 9) from healthy donors in the presence of buffer, heparin, PF4 and IV.3 as indicated. 0.2 IU/mL LMWH lead to stronger generation of procoagulant platelets and completely inhibited by high concentration of unfractionated heparin (UFH). An increase of procoagulant platelets was also observed in presence of PF4, which was inhibited by the Fc-gamma-receptor IIA blocking monoclonal antibody IV.3 (Fig. 8). Further sera from HIPA negative patients were incubated with PRP from healthy volunteers (Fig. 8). Strong increase in procoagulant phenotype was observed in presence of PF4, when treated with 0.2 IU/ml Heparin (LMWH).

Therefore, it was demonstrated that HIT is associated with procoagulant platelets. Furthermore, PF4 can be used as a predictive biomarker for HIT. PF4 enhances the sensitivity of PRP-based flow cytometry assays. The use of Heparin at high concentration as well as monoclonal antibody (IV.3) increases the specificity of these assays.

### 4. Discussion

The increasing number of reports on rare thrombotic events after SARS-CoV-2 vaccination draws public attention and led to concerns regarding the safety of this vaccine due to the uncertainty of the origin of these undesired reactions. To understand the pathophysiology of this phenomenon, the so-called vaccine-induced immune thrombotic thrombocytopenia (VITT), the inventors analyzed sera from 8 patients. The inventors' mostly young, generally fit cohort of patients, presented acutely with atypical thrombosis, primarily, but not exclusively involving the cerebral venous sinuses, an extremely rare manifestation of thrombosis in the general population. All cases developed symptoms within 6-20 days after the ChAdOx1 nCoV-19 vaccination showing a temporal relationship between vaccination and symptoms. The main findings in these cases were thrombocytopenia, high D-dimer, low fibrinogen, and high titer IgG antibodies against PF4 that can induce procoagulant platelet phenotype.

After intensive laboratory investigations of the VITT cases, the inventors were able to identify the serological profile of the pathological antibodies. In a small cohort of vaccinated volunteers, approximately 10% of the individuals developed IgG antibodies against PF4/polyanion complexes within 14 days after the first vaccination; none of them was exposed to heparin in the past 100 days. The inventors observed that IgG binding to PF4 in these sera as well as in VITT sera can be inhibited by heparin but also by increasing the concentration of Spike-RBD. These data may suggest that these antibodies are specific for conformational changes in PF4 that might be induced by negatively charged structures. Of note, no significant IgG binding to platelets was observed in the presence of the vaccine ChAdOx1 nCoV-19. Accordingly, it is very unlikely that Vector (pCDNA4) may be responsible for the high PF4-seroconversion rate in vaccinated individuals. Comparable data were reported earlier. In two very recent reports that appeared while the inventors' application was in preparation. In addition to their observations, the inventors were also able to demonstrate that sera from VITT patients directly induce procoagulant platelets, suggesting a possible mechanism for thrombotic events seen in patients with VITT.

The inventors' data indicate that IgG antibodies against PF4 increase generation of procoagulant platelets in VITT. However, the inventors cannot exclude other co-factor(s) that could also induce thromboembolic complications *in vivo.*

The inventors' study also extends our knowledge on potential therapeutic strategies. First, the increased percentage of procoagulant platelets (CD62p/PS positive) in response to sera from VITT patients *in vitro* appears to represent the central pathomechanism in VITT. Moreover, the inventors' data show also that anticoagulation using non-heparin based therapy, such as argatroban and danaparoid, is safe to treat or to prevent CVST in VITT.

The inventors' study reports on VITT after ChAdOx1 nCoV-19, which is the only SARS-CoV-2 vaccine that includes a simian adenovirus. Disturbances of platelets have been described in association with the intravenous administration of adenovirus gene therapy vectors although it is unclear how that might relate to isolated thrombocytopenia as an adverse event of the vaccine.

Finally, the observed clinical and laboratory features of the VITT are exceptional and rare. Therefore, the value of COVID-19 vaccination to provide critical protection should be considered higher compared to significant health risk of COVID-19. With the better recognition of this rare complication and the availability of efficient therapies, the risk-benefit ratio of ChAdOx1 nCoV-19 might be reconsidered further.

### 5. Conclusion

Although the incidence of VITT after ChAdOx1 nCoV-19 vaccination is very low, the mortality rate is high (37% in our case series). Since a global vaccination campaign is underway and large numbers of people will be vaccinated, an increase in the number of people with this side effect is to be expected, highlighting the importance of a better understanding of the pathophysiology of VITT. In this study, we present immunological and pathological findings in patients with VITT. Furthermore, we show the contribution of antibody-mediated platelet activation in the pathogenesis of VITT.

Finally, the inventors have developed a method which allows or the very first time to reliably and simply determine a predisposition of a subject for the development of thrombocytopenia and/or thromboses or an actual suffering therefrom.

## Claims

**1.** A method for diagnosing a predisposition of a living being to develop thrombocytopenia, comprising the following steps:
1. Providing blood serum of a living being to be diagnosed;
2. Providing thrombocytes of a healthy reference living being;
3. Determining the percentage of thrombocytes of step (2) expressing both of P-selectin and phosphatidylserine on their surfaces to obtain a value Mₚᵣₑ;
4. Incubating an aliquot of said blood serum of step (1) with an aliquot of said thrombocytes of step (2);
5. Determining the percentage of thrombocytes after said incubation in step (4) expressing both of P-selectin and phosphatidylserine on their surfaces to obtain a value Mₚₒₛₜ;
6. Diagnosing a predisposition of said living being to develop thrombocytopenia if Mₚₒₛₜ > Mₚᵣₑ.

**2.** The method of claim 1, **characterized in that** said thrombocytopenia is heparin-induced thrombocytopenia (HIT).

**3.** The method of claim 1, **characterized in that** said thrombocytopenia is vaccine-induced immune thrombocytopenia (VITT).

**4.** The method of claim 3, **characterized in that** said VITT is induced by an anti-SARS-CoV-2 vaccine.

**5.** The method of claim 4, **characterized in that** said anti-SARS-CoV-2 vaccine is a vector-based vaccine, preferably an adenovirus-based or adenovirus-associated-based vector vaccine.

**6.** The method of any of the preceding claims, **characterized in that** said thrombocytes of step (2) are provided as washed thrombocytes.

**7.** The method of any of the preceding claims, **characterized in that** said thrombocytes of step (2) are provided as platelet rich plasma (PRP).

**8.** The method of any of the preceding claims, **characterized in that** in step 4 platelet factor IV (PF4) is added to said incubation mixture of blood serum and thrombocytes.

**9.** The method of any of the preceding claims, **characterized in that** in step 4 heparin is added to said incubation mixture of blood serum and thrombocytes.

**10.** The method of claim 9, **characterized in that** said heparin is low molecular weight heparin (LMWH).

**11.** The method of any of the preceding claims, **characterized in that** determining the percentage of said thrombocytes in step 3 and/or step 5 expressing both of P-selectin and phosphatidylserine on their surfaces is carried out via flow cytometry (FC).

**12.** The method of any of the preceding claims, **characterized in that** in step 6 a predisposition is diagnosed if after said incubation in step (4) ≥ 10% of said thrombocytes have expressed both of P-selectin and phosphatidylserine on their surfaces.

**13.** A method for diagnosing a living of suffering from thrombocytopenia, comprising the following steps:
1. Providing blood serum of a living being to be diagnosed;
2. Providing thrombocytes of a healthy reference living being;
3. Determining the percentage of thrombocytes of step (2) expressing both of P-selectin and phosphatidylserine on their surfaces to obtain a value Mₚᵣₑ;
4. Incubating an aliquot of said blood serum of step (1) with an aliquot of said thrombocytes of step (2);
5. Determining the percentage of thrombocytes after said incubation in step (4) expressing both of P-selectin and phosphatidylserine on their surfaces to obtain a value Mₚₒₛₜ;
6. Diagnosing a predisposition of said living being to develop thrombocytopenia if Mₚₒₛₜ > Mₚᵣₑ.

**13.** A use of a combined presence of P-selectin and phosphatidylserine on the surface of thrombocytes as a diagnostic marker of a predisposition of a living being to develop thrombocytopenia and/or of suffering from thrombocytopenia.

**14.** The use of claim 1, **characterized in that** said thrombocytopenia is heparin-induced thrombocytopenia (HIT) and/or vaccine-induced immune thrombocytopenia (VITT).
